# EUROPEAN PATENT SPECIFICATION

(11) **EP 1 622 664 B1**
(45) Date of publication and mention of the grant of the patent: **27.08.2008**
(21) Application number: 04732132.8
(22) Date of filing: 11.05.2004
(51) Int. Cl.: A61M 11/04

(54) **IMPROVEMENTS RELATING TO VAPORISERS**
VERBESSERUNG BEZÜGLICH VERDAMPFER
PERFECTIONNEMENTS RELATIFS A DES VAPORISATEURS

(30) Priority: 13.05.2003 GB 0310946
(43) Date of publication of application: 08.02.2006
(73) Proprietor: Reckitt Benckiser Healthcare (UK) Limited, Slough, Berkshire SL1 3UH (GB)
(72) Inventor: SHEA-SIMONDS, Duncan Robert, West End, Leicester LE3 0SB (GB)
(74) Representative: O'Brien, Niall James
(86) International application number: PCT/GB2004/002039
(87) International publication number: WO 2004/101039

(56) References cited:
- EP-A- 0 475 902
- WO-A-02/40067
- DE-U- 29 720 104
- US-A- 4 630 775
- US-A- 5 269 460
- US-A- 5 662 835
- US-A- 6 078 728

## Description

This invention relates to a vaporiser, that is to say to a device that disperses a substance into the atmosphere as a vapour, and in particular to a vaporiser that disperses a medicament, such as a decongestant, into the atmosphere by heating.

Vaporisers that disperse substances into the atmosphere as vapour are used for many different purposes. For instance, vaporisers are used to disperse sterilising agents, deodorants and pleasant aromas into the atmosphere, see for example US5269460. A particular application of vaporisers is the dispersal of a medicament into the atmosphere of an enclosed room in order to treat a patient. For example, the patient may be a sleeping child and the medicament may be a decongestant to aid the child's sleep.

Vaporisers typically disperse a substance into the atmosphere by allowing, or causing, a liquid to evaporate so that the vapour enters the atmosphere and disperses. In order to evaporate the liquid, the vaporiser may simply contain a volatile liquid which is allowed to evaporate and the vapour to escape. Alternatively, the vaporiser may include a heater for bringing about or promoting the evaporation of the liquid..

Where the vaporiser includes a heater, a common form of vaporiser is plugged directly into a mains electrical socket, the vaporiser having the general form of a mains electrical plug with an enlarged housing. Such a vaporiser includes a heater and a vaporising chamber, the vaporising chamber containing a reservoir of liquid and including vents through which the vapour produced can escape into the atmosphere. This form of vaporiser is activated by simply plugging the vaporiser into a mains electrical socket and connecting the electricity supply to the heater. The heater causes the liquid to evaporate and the vapour produced exits the vaporiser through the vents.

A common feature of such vaporisers is that the vaporising chamber of the vaporiser may be opened and the liquid in the reservoir replenished.

A major problem with conventional vaporisers that allow replacement of the substance to be dispersed is that the process of replenishing the substance may involve a risk of the substance coming into direct contact with the user's skin and/or being ingested by the user, which may be undesirable or even harmful. Also, because the vaporising chamber needs to be opened in order to replace the substance, a child may be able to open the vaporising chamber and access the substance. This is a particular problem for vaporisers that are connected directly to a mains electrical socket, because the vaporiser will most likely be installed at a location that is readily accessible to the child. In addition, where the substance is a medicament, it may be particularly hazardous for a child to have access to, and possibly ingest, the medicament.

A further problem with many conventional vaporisers is that the vaporising chamber may be opened whilst the vaporiser is connected to the electricity supply. Again, this is particularly hazardous if a child is able to open the vaporising chamber and therefore have access to potentially live components.

There have now been devised improvements to devices for vaporising a substance, which overcome or substantially mitigate the above-mentioned and/or other disadvantages associated with the prior art.

There is provided, a device for vaporising a substance, according to claim 1.

The device is advantageous principally because the reservoir of substance to be vaporised is opened only when the device is closed, thereby reducing or eliminating the risk of direct contact between the substance and the user.

The substance to be vaporised may be any suitable substance including sterilising agents, deodorants, perfumes and medicaments. The invention is particularly useful when used with medicaments for a child, such as decongestants. When a device is used to administer a medicament to a child, the device is typically used for a prolonged period of time (eg overnight) in proximity to an unsupervised child.

The substance to be vaporised may be in any form from which a vapour can be produced. Preferably, however, the substance is in the form of a gel or, more preferably, a liquid.

The device preferably includes a heater in thermal contact with the vaporising chamber. The heater is preferably an electric heater. The device preferably includes a mains electrical plug for connecting the heater to the mains electricity supply. In this case, the device preferably has the general form of a mains electrical plug with an enlarged housing that includes the heater and the vaporising chamber.

The closure is preferably hingedly mounted to the remainder of the device. Most preferably, the chamber is formed between a housing that includes the heater and its associated electrical connections, and the closure. The device preferably includes a plurality of vents which, in this case, are preferably formed in the wall of the closure.

Most preferably, the means for opening the reservoir is a projection extending from an inwardly facing surface of the closure, the projection being adapted to pierce the reservoir on closing the closure. In this case, the reservoir is preferably sealed with a sealing film. Such means for opening the reservoir is advantageous because the reservoir is not opened until it is positioned within the chamber, thereby reducing the likelihood that the substance will be spilled or come into contact with the user's skin.

The device most preferably includes means for preventing unauthorised opening of the closure. As such, the device preferably includes means for retaining the closure in the closed position, said means for retaining being releasable by engagement with a key.

The device comprising a vaporising chamber having a closure, means for receiving a substance to be vaporised, and a vent for allowing vapour to exit the device, the closure being movable between an open position in which a user is able to introduce the substance to be vaporised into the vaporising chamber, and a closed position in which access to the substance within the vaporising chamber is prevented, the vaporiser including means for retaining the closure in the closed position, said means for retaining being releasable by engagement with a key.

The device includes one or more openings into which the key may be inserted and thereby engage the retaining means. Where the device has the form of a mains electrical plug with an enlarged housing that includes the heater and the vaporising chamber, the openings preferably face towards the mains electrical socket into which the device is plugged such that the closure may only be released when the device is unplugged from the socket. Preferably, the retaining means are such that the closure is released by insertion of a key comprising two or more projections into corresponding openings in the device.

Preferably, the retaining means comprises one or more detents mounted in the closure or, more preferably, the housing of the device, and keepers on the other of those components, most preferably on the interior of the closure. The retaining means may have the form of a resilient member with a detent at each end. The projections of the key preferably engage the retaining means with a camming action that displaces the detents inwardly or outwardly, thereby releasing the detents from the keepers.

The substance to be vaporised is supplied in a replaceable cartridge that includes a reservoir of the substance that is, prior to use, sealed. The cartridge includes one or more formations that function as the key for releasing the closure. In such an arrangement, a fresh cartridge can be used to open the device, permitting the removal of a spent cartridge from within the device. The fresh cartridge can be inserted into the device to replace the spent cartridge and the device then closed. Where the closure includes means for opening the reservoir of the fresh cartridge, the action of closing the device opens the reservoir and permits escape of the substance from the reservoir.

The formations that function as a key preferably have the form of one or more projections that extend from the body of the cartridge. Most preferably, the cartridge includes two projections at one end thereof. The two projections are preferably orientated parallel to one another. Preferably, the projections are generally planar and rectangular in shape.

Thus, according to a further aspect of the invention, there is provided a cartridge comprising a cartridge body and a sealed reservoir of a substance to be vaporised, wherein the cartridge body includes one or more formations adapted to engage a device according to the second aspect of the invention, so as to release the closure of said device from the closed position.

The cartridge preferably comprises, in addition to the reservoir of substance to be vaporised, an absorbent pad or the like into which the substance is released when the reservoir is opened and which may draw the substance from the reservoir by capillary action.

The reservoir is preferably sealed, before use, by a sealing membrane. The cartridge is preferably formed so that a projection, eg formed on the closure of the device, may enter the cartridge and pierce the sealing membrane prior to use.

Most preferably, the cartridge comprises a generally planar body, which encloses the absorbent pad, and an enclosure, which encloses the reservoir.

According to a further aspect of the invention, there is provided a kit for vaporising a substance, the kit comprising a device according to the second aspect of the invention and one or more cartridges as described above.

The invention will now be described in greater detail, by way of illustration only, with reference to the accompanying drawings, in which
Figure 1 is a side view of a vaporiser according to the invention;
Figure 2 is a front view of the vaporiser of Figure 1;
Figure 3 is a rear view of the vaporiser;
Figure 4 is a view of the vaporiser from above;
Figure 5 is an exploded view of the vaporiser, from the side and in front;
Figure 6 is an exploded view of the vaporiser, from the side and behind;
Figure 7 is an exploded view of a medicament cartridge according to the invention; and
Figure 8 shows stages in the assembly of the cartridge of Figure 7, from an open condition (a) to the assembled condition (b).

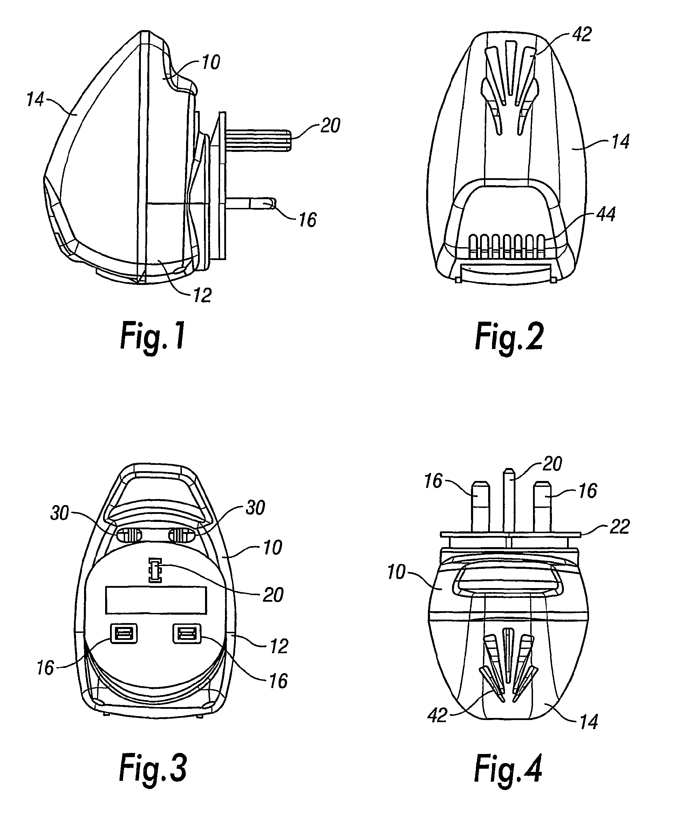

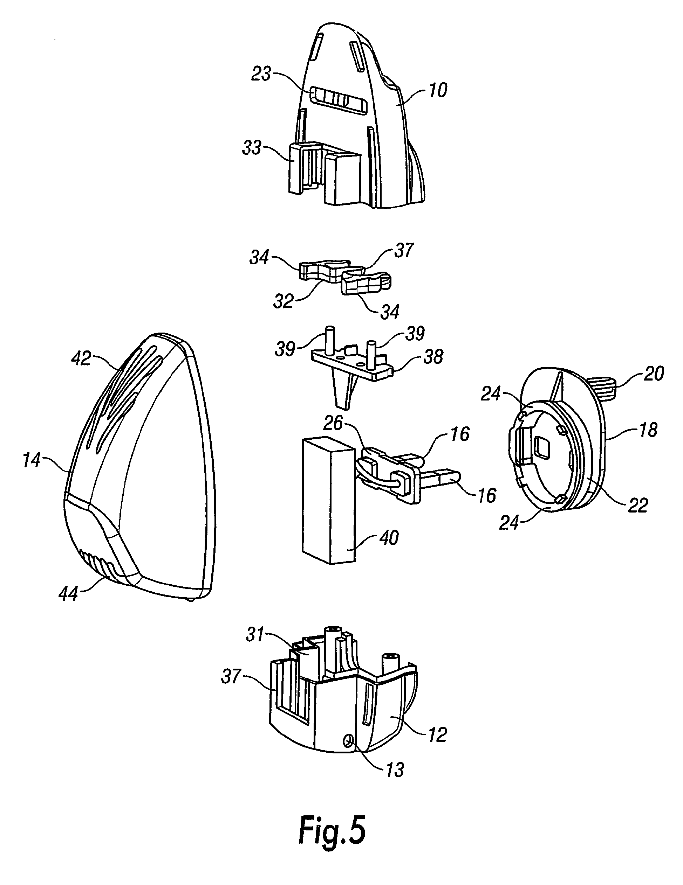

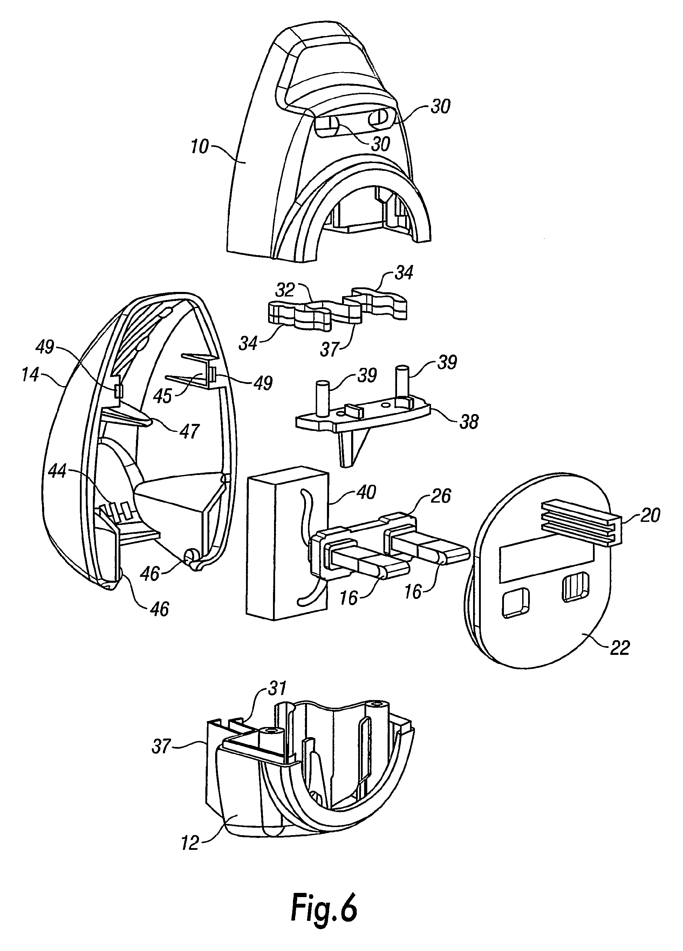

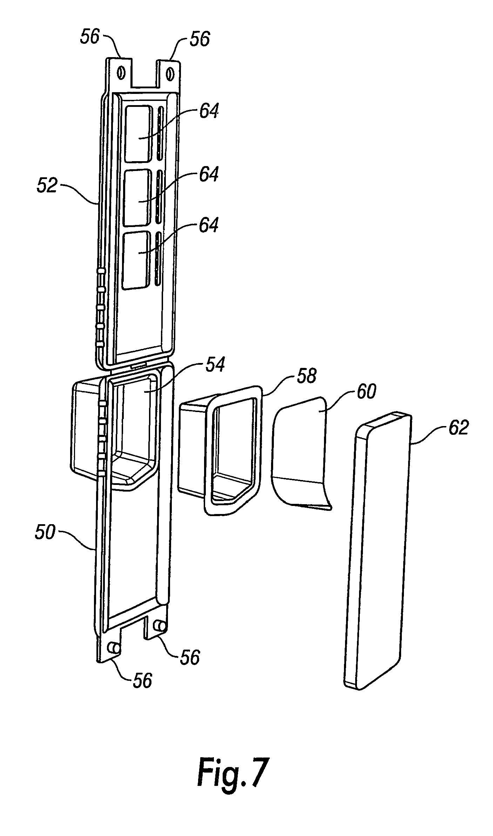

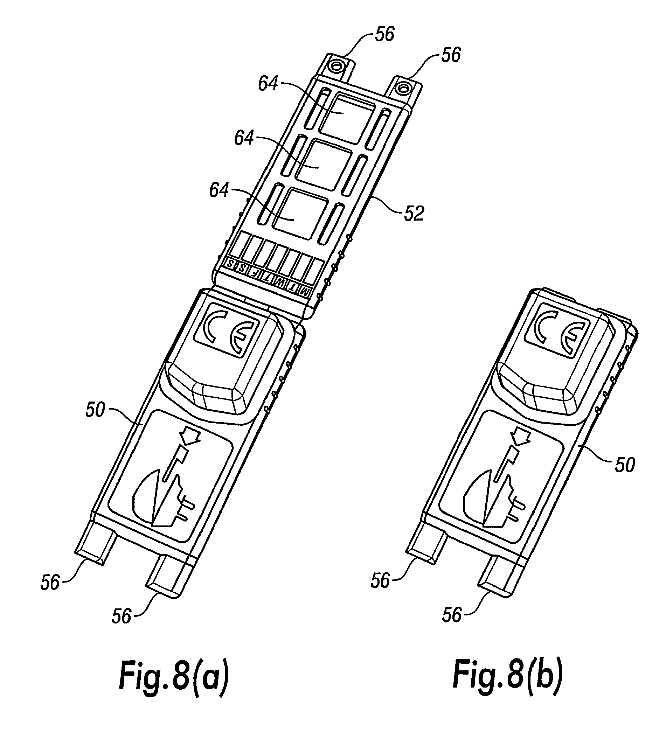

Figures 1 to 6 show a vaporiser according to the invention. As is evident from Figures 1 to 4, the vaporiser has the general form of an electrical plug with an enlarged housing. The structure of the vaporiser is shown more clearly in the exploded views of Figures 5 and 6. The vaporiser comprises an upper housing 10, a lower housing 12, a front cover 14, a heater 40, a lock mounting 38, a locking yoke 32 and plug components 16,18 which together form a plug for engaging with a mains electrical socket. All components of the vaporiser, save for those components that are intended to conduct electricity, are injection moulded in a plastics material. The components of the vaporiser are assembled together to form the vaporiser as shown in Figures 1 to 4. The vaporiser is used in conjunction with the medicament cartridge shown in Figures 7 and 8, and may be sold with such a medicament cartridge, or separately.

The plug comprises a pair of conducting pins 16 of rectangular cross-section that extend through a pair of openings formed in a planar platform 18. The plug further comprises a further pin 20, which is also of rectangular cross-section, orientated parallel to the conducting pins 16 and integrally formed with the platform 18. The pins 16,20 extend perpendicularly from a rear surface of the platform 18 and are arranged as for a standard UK mains electrical plug, with the further pin 20 corresponding to the earth pin of such a plug. The vaporiser is intended for use with a three-pin mains electrical socket, as is standard in the UK. However, it will be clear that the vaporiser may include any suitable means for connecting to an electricity supply.

A connecting formation 22, integrally formed with the platform 18, extends from a front surface of the platform 18 and is adapted to engage the upper and lower housings 10,12. The connecting formation 22 has a short tubular part, and an outwardly extending peripheral flange 24. The short tubular part houses the ends of the conducting pins 16 and the support 26 to which the conducting pins 16 are mounted.

The upper housing 10 has the form of an enclosure which is tapered towards a closed upper end and has an open lower end. The front wall of the upper housing 10 is flat and includes an internal opening 28 of rectangular shape. The rear and side walls of the upper housing 10 have rounded edges and the rear wall has an upwardly facing shoulder towards its upper end. Below the shoulder, the rear wall of the upper housing 10 has a pair of external openings 30 that are approximately in registration with the internal opening 28 in the front wall of the upper housing 10.

A locking yoke 32 is accommodated within the upper housing 10. The locking yoke 32 comprises a pair of clips 34 that are connected by a resilient bridge 37. Each clip is formed with a circular socket within which upstanding lugs 39 of the lock support 38 are received. The clips 34 are therefore capable of limited pivoting movement about the lugs 39. The inwardly-directed ends of the clips 34 project through the internal opening 28 and are formed at their ends with detents that engage keepers 45 on the interior of the front cover 14 as described more fully below. The rearwardly-directed (as viewed in Figure 5) ends of the clips 34 terminate within the upper housing 10 and are accessible through the external openings 30. The arrangement is such that, if the rearward ends of the clips 34 are urged outwards, the forward ends of the clips 34 are urged towards each other.

The lower housing 12 is an enclosure with an open upper end that abuts the open lower end of the upper housing 10. The lower edge of the rear wall of the upper housing 10 and the upper edge of the corresponding rear wall of the lower housing 12 each have semi-circular openings that engage the flange 24 of the connection formation 22. The abutting upper and lower housings 10,12 are affixed together so as to captivate the connecting formation 22 and therefore attach the upper and lower housings 10,12 to the plug.

The front surfaces of the lower and upper housings 12,10 have extensions that together accommodate the heater 40. A further extension on the front of the lower housing 12 constitutes a cartridge holder 37 that accommodates a generally rectangular and planar part of a cartridge adjacent the outer surface of the heater 40. A cartridge may be slid through the open upper end of the holder 37. When the cartridge is engaged with the holder 37, the portion of the cartridge that includes the medicament is situated above the heater 40. The heater 40 is connected by a pair of insulated wires to the support 26 and the conducting pins 16. The heater 40 is therefore activated when the conducting pins 16 are connected to the mains electricity supply.

The front cover 14 has an open rear face, the periphery of which abuts the outer edge of the front surfaces of the upper and lower housings 10,12, as shown in Figure 1 and 2, thereby forming a vaporising chamber. The interior surface of the front cover 14 includes a pair of keepers 45 (one of which is visible in Figure 6) with which the clips 34 engage to secure the front cover 14 in a closed position. The lower edge of the front cover 14 is formed with a pair of inwardly directed studs 46 which engage in corresponding openings 13 in the lower housing 12 to form a hinged connection between the front cover 14 and the lower housing 12. When the clips 34 are disengaged from the keepers, the front cover 14 is movable to an open position in which the upper ends of the upper housing 10 and front cover 14 are separated, thereby allowing the insertion and/or removal of a medicament cartridge.

In order to allow vapour to exit the vaporiser, the front cover 14 has a series of upper vents 42 in its upper wall, and a series of lower vents 44 in its lower front wall. The interior surface of the front wall of the front cover 14 is formed with an inward projection 47 (visible in Figure 6) with a pointed end that extends into the position of the cartridge when the front cover 14 is secured to the upper housing 10.

A cartridge according to the invention is shown in Figures 7 and 8. The cartridge comprises a medicament holder 50 and a cover 52. The medicament holder 50 and cover 52 are of similar dimensions, are generally rectangular and planar in shape, and are hingedly connected at one edge. The upper part (as viewed in Figure 7) of the medicament holder 50 includes a recess 54.

The medicament holder 50 and cover 52 each have a corresponding pair of rectangular extensions at either side of their lower edge (as viewed in Figures 4 and 5) which together form a single pair of extensions 56. Studs on the extensions 56 at the lower edge of the medicament holder 50 engage with an interference fit in corresponding circular openings in the extensions 56 of the cover 52 when the two are brought into engagement by rotation of the cover 52 about its hinged connection to the medicament holder 50.

Figure 7 shows the cartridge in an open state with the additional components that together form a loaded cartridge for use with the vaporiser according to the invention. Those additional components are a medicament capsule 58, which is closed by a sealing film 60, and an absorbent pad 62. The medicament capsule 58 is charged with medicament and sealed by the sealing film 60 affixed to the periphery of the capsule 58. The capsule 58 is received within the recess 54. The absorbent pad 62 is then positioned over the capsule 58, and assembly of the cartridge is completed by folding the cover 52 over the absorbent pad 62 (from the orientation shown in Figure 8(a) to that of Figure 8(b)) and engaging the studs and corresponding openings of the projections 56. The absorbent pad 62 is captivated in the space between the medicament holder 50 and the cover 52. The cover 50 of the cartridge has vents 64 adjacent the absorbent pad 62.

In use, a cartridge is used to open the front cover 14 when the vaporiser is not engaged with a mains electrical socket. The user inserts the pair of extensions 56 of the cartridge into the external openings 30 of the vaporiser. As the extensions 56 are inserted into the external openings 30, the extensions 56 come into contact with inwardly facing surfaces of the rearwardly-directed ends of the clips 34 and urge those ends outwards. This causes the inwardly-directed ends of the clips 34 to pivot inwards and consequently the detents at the ends of the clips 34 disengage from the keepers 45 on the inside of the front cover 14, thereby allowing the front cover 14 of the vaporiser to be separated from the upper housing 10.

Once the upper housing 10 and front cover 14 of the vaporiser are separated, a used cartridge present in the cartridge holder 37 is removed. A fresh cartridge is then inserted through the open upper end of the cartridge holder 37. The cartridge is inserted so that the extensions 56 enter the holder 38 first and in an orientation such that, once in position, the medicament capsule 58 is situated above the heater 40 and the vents 64 face the interior of the front cover 14.

Once the loaded cartridge has been positioned within the cartridge holder 38, the front cover 14 is closed by pivoting the front cover 14 into abutment with the upper and lower housings 10,12. The detents at the ends of the clips 34 then engage behind the keepers 45, thereby securing the upper housing 10 and front cover 14 together. In addition, lugs 49 that extend from the front surfaces of the keepers 45 locate within the ends of the internal opening 28. On closing of the front cover 14, the pointed end of the inward projection 47 of the front cover 14 will enter the loaded cartridge through one of the vents 64, pierce the absorbent pad 62 and pierce the sealing film 60.

Once the front cover 14 is closed and the sealing film 60 has been pierced by the inward projection, liquid medicament will be drawn into the absorbent pad 62. The vaporiser is then plugged into a mains electrical socket so that power is supplied to the heater 40. As the heater 40 heats the cartridge and the absorbent pad 62, the liquid medicament will slowly vaporise and the vapour will pass through the vents 64 in the cartridge into the interior of the front cover 14. The vapour will then pass through the upper and lower vents 42,44 in the front cover 14 into the atmosphere of the room in which the vaporiser has been placed. Typically, the patient will be asleep in this room and the vapour will be inhaled by the patient whilst asleep.

After use, the vaporiser may be removed from the electrical socket into which it was plugged, or simply switched off. Once the cartridge has been fully exhausted, the vaporiser is unplugged from the mains socket and a fresh cartridge is used to open the front cover 14. The exhaused cartridge is removed and replaced by the fresh cartridge.

## Claims

1. A device for vaporising a substance, the device comprising a vaporising chamber having a closure (14) means (37) for receiving a reservoir of a substance to be vaporised, and a vent (42) for allowing vapour to exit the device, the closure (14) being movable between an open position in which a user is able to introduce the reservoir (58,60) of substance to be vaporised into the vaporising chamber, and a closed position, wherein the closure is provided with means (47) for opening the reservoir (50,52) such that the reservoir is opened by the action of moving the closure from the open position to the closed position, and the device includes means (34) for retaining the closure in the closed position, said means (34) for retaining being releasable by engagement with a key, the device further comprising a replaceable cartridge (50,52) that includes a sealed reservoir (58,60) of the substance that is to be vaporised, wherein the cartridge (50,52) includes one or more formations (56) that function as the key for releasing the closure.

2. A device as claimed in Claim 1, wherein the device further comprises a heater in thermal contact with the vaporising chamber.

3. A device as claimed in Claim 2, wherein the heater is an electric heater.

4. A device as claimed in Claim 3, wherein the device has the general form of a mains electrical plug with an enlarged housing that includes the heater and the vaporising chamber.

5. A device as claimed in any preceding claim, wherein the means for opening the reservoir comprises a projection extending from an inwardly facing surface of the closure, the projection being adapted to pierce the reservoir on moving the closure to the closed position.

6. A device as claimed in any preceding claim, wherein the closure is hingedly connected to the remainder of the device.

7. A device as claimed in Claim 1, wherein the device includes one or more openings into which the key may be inserted to engage the retaining means.

8. A device as claimed in Claim 7, wherein the retaining means comprises one or more detents mounted in the closure or a housing of the device, and keepers on the other of those components.

9. A device as claimed in Claim 8, wherein the detents are mounted in the housing and the keepers on the interior of the closure.

10. A device as claimed in Claim 8 or Claim 9, wherein the retaining means comprises a pair of detents mounted at each end of a resilient member.

11. A device as claimed in Claim 10, wherein the key engages the retaining means with a camming action that displaces the detents inwardly or outwardly, thereby releasing the detents from the keepers.

12. A device as claimed in Claim 1, wherein the formations that function as a key have the form of one or more projections that extend from the body of the cartridge.

13. A cartridge comprising a cartridge body (50,52) and a sealed reservoir (58,60) of a substance to be vaporised, wherein the cartridge body (50,52) includes one or more formations (56) adapted to engage a device according to Claim 1 so as to release the closure of said device from the closed position.

14. A cartridge as claimed in Claim 13, wherein the cartridge further comprises an absorbent pad or the like into which the substance is released when the reservoir is opened.

15. A cartridge as claimed in Claim 13 or Claim 14, wherein the reservoir is sealed by a sealing membrane.

16. A kit for vaporising a substance, the kit comprising a device according to Claim 1 and one or more cartridges according to any one of Claims 13 to 15.

## Patentansprüche

1. Vorrichtung zum Verdampfen einer Substanz, wobei die Vorrichtung Folgendes umfasst: eine Verdampfungskammer mit einem Verschluss (14), ein Mittel (37) zum Aufnehmen eines Behälters einer zu verdampfenden Substanz und eine Belüftung (42) zum Ermöglichen des Austretens von Dampf aus der Vorrichtung, wobei der Verschluss (14) zwischen einer offenen Position, in welcher der Anwender in der Lage ist, den Behälter (58, 60) einer zu verdampfenden Substanz in eine Verdampfungskammer einzubringen, und einer geschlossenen Position bewegbar ist, wobei der Verschluss mit Mittel (47) zum Öffnen des Behälters (50, 52) derart, dass der Behälter durch die Wirkung der Bewegung des Verschlusses von der offenen Position zu der geschlossenen Position geöffnet wird, versehen ist und die Vorrichtung ein Mittel (34) zum Halten des Verschlusses in der geschlossenen Position einschließt, wobei das Mittel (34) zum Halten durch Eingriff mit einem Schlüssel lösbar ist, wobei die Vorrichtung ferner eine ersetzbare Patrone (50, 52) umfasst, die einen abgedichteten Behälter (58, 60) der zu verdampfenden Substanz einschließt, wobei die Patrone (50, 52) eine oder mehrere Gebilde (56) als Schlüssel zum Lösen des Verschlusses einschließt.

2. Vorrichtung nach Anspruch 1, wobei die Vorrichtung ferner einen Heizer in thermischem Kontakt mit der Verdampfungskammer umfasst.

3. Vorrichtung nach Anspruch 2, wobei der Heizer ein elektrischer Heizer ist.

4. Vorrichtung nach Anspruch 3, wobei die Vorrichtung die allgemeine Form eines elektrischen Netzsteckers mit einem vergrößerten Gehäuse, das den Heizer und die Verdampfungskammer einschließt, umfasst.

5. Vorrichtung nach einem der vorangehenden Ansprüche, wobei das Mittel zum Öffnen des Behälters einen Vorsprung umfasst, der sich von einer nach innen gewandten Oberfläche des Verschlusses erstreckt, wobei der Vorsprung derart angepasst ist, den Behälter beim bewegen des Verschlusses zur geschlossenen Position zu durchstechen.

6. Vorrichtung nach einem der vorangehenden Ansprüche, wobei der Verschluss schwenkbar mit dem Rest der Vorrichtung verbunden ist.

7. Vorrichtung nach Anspruch 1, wobei die Vorrichtung eine oder mehrere Öffnungen einschließt, in welche der Schlüssel eingeführt werden kann, um in das Haltemittel einzugreifen.

8. Vorrichtung nach Anspruch 7, wobei das Haltemittel eine oder mehrere im Verschluss oder einem Gehäuse der Vorrichtung angebrachte Feststellvorrichtungen und Anker am anderen dieser Bestandteile umfasst.

9. Vorrichtung nach Anspruch 8, wobei die Feststellvorrichtungen im Gehäuse und die Anker am Inneren des Verschlusses angebracht sind.

10. Vorrichtung nach Anspruch 8 oder Anspruch 9, wobei das Haltemittel ein Paar Feststellvorrichtungen umfasst, die an jedem Ende eines Federelements angebracht sind.

11. Vorrichtung nach Anspruch 10, wobei der Schlüssel in das Haltelement mit einer Schiebewirkung eingreift, die die Feststellvorrichtungen nach innen oder außen verschiebt, wodurch die Feststellvorrichtungen aus den Ankern gelöst werden.

12. Vorrichtung nach Anspruch 1, wobei die Gebilde, die als Schlüssel fungieren, die Form von einem oder mehreren Vorsprüngen aufweisen, die sich vom Körper der Patrone erstrecken.

13. Patrone, umfassend einen Patronenkörper (50, 52) und einen angedichteten Behälter (58, 60) einer zu verdampfenden Substanz, wobei der Patronenkörper (50, 52) eine oder mehrere Gebilde (56) einschließt, die zum derartigen Eingriff in eine Vorrichtung nach Anspruch 1 angepasst sind, dass der Verschluss der Vorrichtung aus der geschlossenen Position gelöst wird.

14. Patrone nach Anspruch 13, wobei die Patrone ferner ein Absorptionskissen oder dergleichen umfasst, in welches die Substanz freigesetzt wird, wenn der Behälter geöffnet ist.

15. Patrone nach Anspruch 13 oder Anspruch 14, wobei der Behälter durch eine Dichtungsmembran abgedichtet ist.

16. Bausatz zum Verdampfen einer Substanz, wobei der Bausatz eine Vorrichtung nach Anspruch 1 und eine oder mehrere Patronen nach einem der Ansprüche 13 bis 15 umfasst.

## Revendications

1. Dispositif destiné à vaporiser une substance, le dispositif comprenant une chambre de vaporisation comportant une enceinte (14), un moyen (37) destiné à recevoir un réservoir d'une substance devant être vaporisée, et une mise à l'air libre (42) destinée à laisser la vapeur quitter le dispositif, l'enceinte (14) étant mobile entre une position ouverte dans laquelle un utilisateur est capable d'introduire le réservoir (58, 60) d'une substance devant être vaporisée à l'intérieur de la chambre de vaporisation, et une position fermée dans laquelle l'enceinte est dotée d'un moyen (47) destiné à ouvrir le réservoir (50, 52) de telle sorte que le réservoir est ouvert par l'action de déplacement de l'enceinte de la position ouverte à la position fermée, et le dispositif comprend un moyen (34) destiné à maintenir l'enceinte dans la position fermée, ledit moyen (34) de retenue pouvant être libéré par un engagement avec une clé, le dispositif comprenant en outre une cartouche remplaçable (50, 52) qui comprend un réservoir scellé (58, 60) de la substance qui doit être vaporisée, où la cartouche (50, 52) comprend une ou plusieurs formations (56) qui fonctionnent comme la clé destinée à libérer l'enceinte.

2. Dispositif selon la revendication 1, lequel dispositif comprend en outre un dispositif chauffant en contact thermique avec la chambre de vaporisation.

3. Dispositif selon la revendication 2, dans lequel le dispositif chauffant est un dispositif chauffant électrique.

4. Dispositif selon la revendication 3, lequel dispositif présente la forme générale d'une prise électrique de courant avec un boîtier agrandi qui comprend le dispositif chauffant et la chambre de vaporisation.

5. Dispositif selon l'une quelconque des revendications précédentes, dans lequel le moyen destiné à ouvrir le réservoir comprend une protubérance s'étendant depuis une surface orientée vers l'intérieur de l'enceinte, la protubérance étant conçue pour percer le réservoir lors du déplacement de l'enceinte vers la position fermée.

6. Dispositif selon l'une quelconque des revendications précédentes, dans lequel l'enceinte est reliée par articulation au reste du dispositif.

7. Dispositif selon la revendication 1, lequel dispositif comprend une ou plusieurs ouvertures dans lesquelles la clé peut être insérée pour s'engager avec le moyen de retenue.

8. Dispositif selon la revendication 7, dans lequel le moyen de retenue comprend un ou plusieurs éléments d'encliquetage montés dans l'enceinte ou un boîtier du dispositif et des éléments de maintien sur l'autre de ces composants.

9. Dispositif selon la revendication 8, dans lequel les éléments d'encliquetage sont montés dans le boîtier et les éléments de maintien sont montés à l'intérieur de l'enceinte.

10. Dispositif selon la revendication 8 ou la revendication 9, dans lequel le moyen de retenue comprend une paire d'éléments d'encliquetage montés à chaque extrémité d'un élément élastique.

11. Dispositif selon la revendication 10, dans lequel la clé s'engage avec le moyen de retenue à l'aide d'une action de came qui déplace les éléments d'encliquetage vers l'intérieur ou vers l'extérieur, en libérant de cette manière les éléments d'encliquetage des éléments de maintien.

12. Dispositif selon la revendication 1, dans lequel les formations qui fonctionnent comme une clé présentent la forme d'une ou plusieurs protubérances qui s'étendent depuis le corps de la cartouche.

13. Cartouche comprenant un corps de cartouche (50, 52) et un réservoir scellé (58, 60) d'une substance devant être vaporisée, où le corps de cartouche (50, 52) comprend une ou plusieurs formations (56) conçues pour s'engager avec un dispositif selon la revendication 1 de façon à libérer l'enceinte dudit dispositif de la position fermée.

14. Cartouche selon la revendication 13, laquelle cartouche comprend en outre un tampon absorbant ou analogue dans lequel la substance est libérée lorsque le réservoir est ouvert.

15. Cartouche selon la revendication 13 ou la revendication 14, dans laquelle le réservoir est scellé par une membrane d'étanchéité.

16. Kit destiné à vaporiser une substance, le kit comprenant un dispositif selon la revendication 1 et une ou plusieurs cartouches selon l'une quelconque des revendications 13 à 15.
